# EUROPÄISCHE PATENTSCHRIFT

(11) **EP 2 093 308 B1**
(45) Veröffentlichungstag und Bekanntmachung des Hinweises auf die Patenterteilung: **11.09.2013**
(21) Anmeldenummer: 09001160.2
(22) Anmeldetag: 28.01.2009
(51) Int. Cl.: C23C 22/02, C23C 22/57, C23C 22/76, C23C 22/77, C23C 22/78

(54) **Verfahren zum Behandeln eines Magnesiumsbauteils**
Method of treating a magnesium component
Procédé de traitement d'un composant de magnésium

(30) Priorität: 13.02.2008 DE 102008009069
(43) Veröffentlichungstag der Anmeldung: 26.08.2009
(73) Patentinhaber: Helmholtz-Zentrum Geesthacht Zentrum für Material- und Küstenforschung GmbH, 21502 Geesthacht (DE)
(72) Erfinder: Scharnagl, Nico, 21514 Büchen (DE); Blawert, Carsten, 21481 Lauenburg (DE)
(74) Vertreter: Grebner, Christian Georg Rudolf

(56) Entgegenhaltungen:
- WO-A-2006/108655
- US-A1- 2004 039 096
- US-A1- 2004 092 329
- US-A1- 2007 224 244

## Beschreibung

Die Erfindung betrifft ein Verfahren zum Behandeln eines Werkstücks aus Magnesium oder einer Magnesiumlegierung.

Es ist bekannt, dass Magnesium und seine Legierungen leichte und unedle metallische Konstruktionswerkstoffe sind. Daher neigen Magnesium und die Legierungen daraus sehr stark zu Kontaktkorrosion.

Das Korrosionsverhalten von Magnesium bzw. Magnesiumoberflächen kann durch Konversions- bzw. Reaktionsschichten und durch anorganische oder organische Beschichtungen modifiziert werden. Beispielsweise werden bei Verfahren, bei denen in einem Elektrolytplasma eine anodische Oxidation oder Substratoberfläche erfolgt, feste und dichte Schichten aus Magnesiumoxiden und/oder -phosphaten mit elektrischer Isolationswirkung und einem guten Verschleißwiderstand hergestellt. Jedoch erfordern diese Schichten in der Regel zusätzlich eine Versiegelung durch eine organische Beschichtung (Top-Coat), um einen Langzeit-Korrosionsschutz zu gewährleisten. Außerdem sind diese Verfahren in der Regel vergleichsweise kostspielig.

Magnesium besitzt zwar an Luft einen guten Korrosionswiderstand, ist jedoch in Chlorid-, Sulfat-, Karbonat- und Nitrat-haltigen Lösungen nicht stabil. Magnesiumlegierungen bilden erst bei pH-Werten über 11 stabile Deckschichten, so dass für den technisch relevanten pH-Bereich zwischen 4,5 und 8,5, in dem z.B. Aluminium stabile Deckschichten entwickelt, keine wirksamen und bei Beschädigungen von selbst ausheilenden Schutzschichten existieren.

Zudem ist Magnesium der unedelste Konstruktionswerkstoff, so dass es einerseits zu starker Auflösung in Folge mikrogalvanischer Korrosion neigt, insbesondere hervorgerufen durch Fe-, Ni- und Cohaltige Verunreinigungen und andererseits bei Magnesiumlegierungen zu interner galvanischer Korrosion durch eine zweite edlere Phase oder Einschlüsse kommt. Da Magnesium häufig im Verbund mit edleren Werkstoffen eingesetzt wird, ist bei Anwendungen in aggressiven Medien und/oder der Anwesenheit von Wasser eine Beschichtung der Bauteile zur Vermeidung der Kontaktkorrosion unerlässlich.

Das Korrosions- und Verschleißverhalten von Magnesiumoberflächen kann, je nach Verwendung und Einsatz, durch Konversions- bzw. Reaktionsschichten und durch anorganische oder auch organische Beschichtungen modifiziert werden.

Beispielsweise sind in US 2006/0063872 A1, EP 0 949 353 B1, US 2005/0067057 A1, US 4,973,393, US 5,993,567, WO 99/02759 A1, und DE 199 13 242 C2 zahlreiche Verfahren oder Maßnahmen zum Korrosionsschutz von Magnesium und seinen Legierungen beschrieben.

In US-A-2007/0224244 sind allgemein korrosionsbeständige Beschichtungen für biologisch abbaubare, metallische Implantate beschrieben, wobei eine implantierbare medizinische Vorrichtung eine bioabbaubare metallische Region aufweist, auf der eine korrosionsbeständige Polymerschicht aufgetragen ist.

Ferner offenbart WO-A-2006/108 655 ein Verfahren zur Bildung einer sichtbaren Nicht-Chromat-Korrosionsbeschichtung für Magnesium und Magnesiumlegierungen, wobei eine wässrige Prozesslösung vorgesehen ist, die eine Fluorsilikonsäure und ein wasserlösliches pH-Einstellungsmittel aufweist.

Ausgehend von diesem Stand der Technik besteht die Aufgabe der Erfindung darin, eine günstige und einfache korrosionsbeständige sowie nicht toxische Beschichtung für Bauteile aus Magnesium oder Magnesiumlegierungen bzw. für Werkstücke aus Magnesium oder mit Magnesium-haltigen Oberflächen bereitzustellen, wobei die Ausbildung der Beschichtung auf der Oberfläche des Werkstücks möglichst einfach sein soll.

Gelöst wird diese Aufgabe durch ein Verfahren zum Behandeln eines Werkstücks aus Magnesium oder einer Magnesiumlegierung, das dadurch weitergebildet wird, dass das Werkstück, insbesondere eine oder die Oberfläche des Werkstücks, mit einer Polymerlösung benetzt oder versehen wird, wobei die Polymerlösung Polyetherimid und ein Lösemittel enthält,
wobei als ein mit Wasser mischbares Lösemittel Dimethylacetamid (DMAc) und/oder Dimethylformamid (DMF) und/oder N-Methylpyrrolidon (NMP) und/oder gamma-Butyrolacton (GBL) oder wobei als ein mit Wasser nicht-mischbares Lösemittel Dichlormethan und/oder Chloroform und/oder 1,2-Dichlorethan eingesetzt wird,
wobei nach Trocknung des Werkstücks, vorzugsweise nach Herausziehen des Werkstücks aus der oder nach Benetzung des Werkstücks mit der Polymerlösung, eine korrosionsbeständige dichte, nicht poröse oder porenfreie Beschichtungslage auf der Oberfläche des Werkstücks ausgebildet ist oder wird.

Hierbei ist oder wird in Abhängigkeit, insbesondere des Anteils und/oder der Art, des Lösemittels, vorzugsweise in Abhängigkeit der Art des Lösemittels in der Polymerlösung, nach Trocknung des Werkstücks, vorzugsweise nach Herausziehen des Werkstücks aus der oder nach Benetzung des Werkstücks mit der Polymerlösung, eine korrosionsbeständige dichte, nicht poröse, porenfreie oder poröse Beschichtungslage auf der Oberfläche des Werkstücks ausgebildet.

Die Erfindung beruht auf dem Gedanken, dass durch eine Polyetherimid-haltige Polymerlösung und durch die Art des Lösemittels und/oder des Anteils in der Polymerlösung eine poröse oder nicht poröse Beschichtung für Bauteile oder Werkstücke aus Magnesium auf der Oberfläche des Bauteils bzw. des Werkstücks ausgebildet wird. Durch das vorgeschlagene Verfahren wird ein einfaches, weniger aufwändiges Beschichtungsverfahren vorgeschlagen, das zudem preisgünstig ist. Die Bauteile bzw. Werkstücke sind aus einem Magnesiumwerkstoff hergestellt, so dass durch die ausgebildete bzw. aufgetragene Beschichtungslage ein blutverträglicher und dauerhafter sowie belastungsfähiger Korrosionsschutz für das Werkstück bzw. für Magnesium oder dessen Legierung bereitgestellt wird.

Dadurch, dass eine Beschichtungslage aus Polyetherimid auf dem Werkstück aufgebracht wird, wird ein Korrosionsschutz der Oberfläche des Werkstücks dauerhaft erreicht, wodurch ein langzeitstabiler und blutverträglicher bzw. blutkompatibler Korrosionsschutz gegeben ist. Hierdurch werden die Werkstücke gegen mechanische und korrosive Angriffe durch die widerstandsfähige Polymerschicht passiviert bzw. geschützt. Insbesondere wird das Einbringen von Wasser sowie anderen, die Korrosion fördernden oder korrosiv wirkenden Substanzen stark herabgesetzt oder verhindert. Bei der Beschichtung entstehen in der Regel keine oder nur geringfügige Mengen an umweltschädlichen Substanzen.

Beispielsweise sind Polyetherimide unter den Bezeichnungen "UL-TEM" (von General Electric) oder "RAU-PEI" von Rehau bekannt.

Im Rahmen der Erfindung ist es durch die Aufbringung einer korrosionsbeständigen Beschichtung auf die Oberfläche(n) von Werkstücken möglich, die Porosität von bereits auf das Werkstück aufgebrachten porösen Schutzschichten herabzusetzen, wobei insbesondere die Schutzschichten durch die Polyetherimid-Beschichtung versiegelt wird.

Die für die Ausbildung von korrosionsbeständigen Beschichtungen auf einem Werkstück verwendeten Polyetherimide betreffen ausschließlich die Polymerklasse der Polyetherimide, wobei es im Rahmen der Erfindung möglich ist, dass mehrere, verschiedene Polyethermimide in der Polymerlösung vorhanden sind.

Ferner gestattet es das erfindungsgemäße Verfahren, die Oberfläche oder Defekte von an der Oberfläche beschädigten Werkstücken, die mit einer Korrosionsbeschichtung (bereits) versehen sind, auf einfache Weise zu heilen oder zu reparieren, im dem auf die Werkstück-Oberfläche eine Polyetherimid-Polymerlösung, z.B. durch Eintauchen in eine Polymerlösung oder durch Aufsprühen der Polymerlösung, aufgetragen oder aufgebracht wird.

Darüber hinaus zeichnet sich die aufgebrachte Polyetherimid-beschichtung durch eine besonders gute Haftung auf den Magnesium-Werkstücken bzw. auf deren Oberflächen aus. Die Beschaffenheit der Beschichtung wird über eine entsprechende Zusammensetzung der Polymerlösung und die Wahl des Beschichtungsverfahrens gesteuert.

Die Porosität der Beschichtungslage ist abhängig von der Verwendung eines Lösemittels bzw. dessen Eigenschaften und dessen Konzentration in der Polymerlösung. Beispielsweise wird eine Lösung aus einem nicht mit Wasser mischbaren Lösemittel wie Dichlormethan und einer hohen Konzentration an Polyetherimid, z.B. größer als 3 Gew.-%, zu einer Beschichtungslage auf dem Wirkstoff mit einer geringen oder keiner Porosität führen, so dass niedrige Korrosionsraten erreicht werden. Überdies ist es aufgrund der aufgebrachten Polyetherimid-Beschichtung(en) auf den Werkstücken oder Bauteilen, die Korrosion bzw. die Korrosionsraten an den Werkstücken oder Bauteilen gezielt einzu-zustellen oder zu überwachen.

Vorzugsweise weist eine erfindungsgemäß beschichtete (reine) Magnesiumlegierung eine Korrossionsrate unter 1,0 mm / Jahr, vorzugsweise unter 0,9 oder 0,8 mm / Jahr auf, gemessen mittels eines Salzsprühtests nach DIN 50021.

Im Gegensatz dazu führt eine Beschichtungslage mit einer Lösung aus wassermischbaren Lösemitteln und einer niedrigen Konzentration des Polyetherimid, z.B. von unter 10 Gew.-%, zu einer hohen bzw. erhöhten Porosität und damit zu einer im Vergleich zur nicht porösen Beschichtungslage zu einer höheren Korrosionsrate.

Durch das erfindungsgemäße Verfahren wird zudem eine korrosionsbeständige Beschichtung von Magnesium-Werkstücken oder Magnesiumlegierungen mittels eines Polyetherimid auch eine nachträgliche chemische Veränderung der Oberfläche durch geeignete Reagenzien oder Verfahren ermöglicht. Im Rahmen der Erfindung werden dabei Polymere der Stoffklasse der Polyetherimide eingesetzt. Durch die Oberflächenmodifikation des Werkstückes, z.B. durch chemische Reaktionen oder Plasmabehandlung usw., wird auch eine bessere Blutverträglichkeit in einer medizinischen Anwendung des Werkstücks, beispielsweise als Implantat, erreicht.

Insbesondere wird das Werkstück in die Polymerlösung eingetaucht oder mit der Polymerlösung besprüht, so dass die Oberfläche des Werkstücks mit der Polymerlösung versehen wird.

Darüber hinaus zeichnet sich das Verfahren dadurch aus, dass die Oberfläche des Werkstücks vor dem Benetzen gereinigt wird oder ist. Alternativ ist die Oberfläche des zu beschichtenden Werkstücks vor dem Benetzen ungereinigt. Ferner wird die Dicke der Beschichtungslage auf der Oberfläche des Werkstücks dadurch erhöht und so kontrolliert, indem nach einem Benetzungs- bzw. Tauchvorgang und Trocknungsvorgang das bereits beschichtete Werkstück wieder in eine Polymerlösung mit Polyetherimid eingetaucht wird. Durch Wiederholungen des Benetzungsvorgang (Eintauchen oder Aufsprühen) und des Trocknungsvorgangs wird sukzessive die Beschichtungsdicke auf der Oberfläche des Werkstücks erhöht.

Gemäß einer bevorzugten Ausgestaltung des Verfahrens ist vorgesehen, dass vor dem Benetzen mit der Polyetherimid-Polymerlösung die Oberfläche des Werkstücks mit einer Konversionsschicht oder einer Haftschicht versehen wird oder ist. Dadurch dass die Werkstückoberfläche in einem Verfahrensschritt vorbehandelt wird, ergeben sich beim Benetzen der Oberfläche aufgrund der erzielten bzw. ausgebildeten Konversionsschichten oder Haftschichten verbesserte Eigenschaften der zu benetzenden Werkstückoberfläche. Unter dem Begriff "Konversionsschicht" wird beispielsweise eine Schicht verstanden, die durch chemische Umwandlung (Konversion) der metallischen Oberfläche und verschiedenen Bestandteilen von Elektrolyten oder dergleichen gebildet wird. Durch eine auf der Werkstückoberfläche aufgebrachte Haftschicht wird die Haftung der anschließend aufgebrachten korrosionsbeständigen Schicht bzw. Korrosionsschicht verbessert. Im Rahmen der Erfindung kann eine Konversionsschicht identisch mit einer Haftschicht sein.

Darüber hinaus zeichnet sich das Verfahren dadurch aus, dass als Lösemittel ein mit Wasser mischbares Lösemittel nach Anspruch 1 eingesetzt wird, so dass eine poröse Beschichtung auf dem Werkstück ausgebildet wird. Insbesondere lassen sich poröse Beschichtungslagen auf dem Werkstück dadurch ausbilden, dass die Konzentration von Polyetherimid in der Polymerlösung unterhalb von 10 Gew.-% liegt.

Als mit Wasser mischbare Lösemittel werden Dimethylacetamid (DMAc) und /oder Dimethylformamid (DMF) und/oder N-Methylpyrrolidon (NMP) und/oder gamma-Butyrolacton (GBL) eingesetzt.

Gemäß einer alternativen Ausführungsform wird als Lösemittel ein mit Wasser nicht mischbares Lösemittel nach Anspruch 1 eingesetzt, so dass eine dichte, porenfreie Beschichtung auf dem Werkstück gebildet wird. Hierbei wird als Lösemittel Dichlormethan und/oder Chloroform und/oder 1,2-Dichlorethan eingesetzt.

Gemäß einer Weiterbildung des Verfahrens ist vorgesehen, dass die Konzentration von Polyetherimid in der Polymerlösung zwischen 0,5 Gew.-% bis 20 Gew.-% beträgt.

Bei der Ausbildung einer porösen Beschichtungslage ist außerdem vorgesehen, dass die Durchmesser der Poren bei der porösen Beschichtungslage auf dem Werkstück zwischen 10 nm bis 10 µm, insbesondere 2 µm, betragen.

Überdies zeichnet sich das Verfahren dadurch aus, dass die aufgebrachte Beschichtungslage auf dem Werkstück derart ausgebildet ist oder wird, dass das Werkstück bei einer mechanischen Krafteinwirkung auf das Werkstück an der Stelle der Krafteinwirkung durch die Beschichtungslage korrosionsbeständig ist oder gegen Korrosion geschützt bleibt, wodurch die beschädigte Stelle durch die aufgetragene Beschichtung im Hinblick auf die Korrosion unverändert geschützt bleibt. Bei einer mechanischen Krafteinwirkung auf die Oberfläche wird bei einer Beschädigung auch die aufgebrachte Beschichtungslage ebenfalls beeinträchtigt bzw. geschädigt, wobei die beschädigte Stelle trotzdem weiterhin korrosionsgeschützt bleibt, wodurch auch an der beschädigten Stelle die niedrige Korrosionsrate aufgrund der Beschichtung erhalten ist.

Bevorzugterweise wird die Polymerlösung auf ein mit einer defekten Korrosionsschicht versehenes Werkstück an dessen Oberfläche aufgetragen bzw. aufgebracht sowie anschließend getrocknet, so dass die defekte Stelle der Korrosionsschicht mit einer Beschichtungslage oder Beschichtung versehen wird. Somit wird auf eine einfache Weise eine schnelle und effektive Reparatur von Defekten oder schadhaften Stellen auf der Werkstückoberfläche möglich

Darüber hinaus zeichnet sich das Verfahren dadurch aus, dass die Haftung der Beschichtungslage größer als die Haftung einer Beschichtungslage aus Acrylat, insbesondere bei gleicher Beschichtungsdicke, ist. Es hat sich in Versuchen gezeigt, dass die aufgebrachte Beschichtungslage eine starke Haftung auf der Magnesiumoberfläche des Werkstücks hat, wodurch auch bei einer mechanischen Belastung der Oberfläche das Bauteil bzw. das Werkstück korrosionsbeständig bleibt. Die Beschichtungslage wird somit auch bei mechanischen Belastungen auf der Oberfläche des Werkstücks verbleiben.

Weiterhin zeichnet sich das Verfahren dadurch aus, dass die Polymerlösung Partikel oder Inhibitoren oder therapeutische oder medikamentöse Wirkstoffe enthält, so dass Partikel oder Inhibitoren oder therapeutische oder medikamentöse Wirkstoffe in die Beschichtungslage eingebaut oder eingebracht oder aufgebracht werden. Hierdurch ergibt sich eine Reihe von zahlreichen Anwendungen eines beschichteten Werkstücks beispielsweise in der Medizintechnik als Implantat oder als vaskuläre Stützeinrichtung.

In Abhängigkeit der Konzentration und auch der Verweildauer des Werkstücks in der Polymerlösung bei der Behandlung lässt sich gezielt eine vorbestimmte Schichtdicke der aufgebrachten Beschichtung einstellen. Insbesondere beträgt die Schichtdicke zwischen 1 µm bis 100 µm, um eine besonders hohe korrosionsbeständige Beschichtungslage auszubilden. Beispielsweise haben Versuche mit nur einem Tauchgang in einer 2%igen Dichlormethanlösung zu einer 5 µm dicken Beschichtungslage geführt, während bei mehrfachem Tauchen ein Vielfaches der Schichtdicke erzielt wurde.

Bei den Versuchen wurden die trockenen Werkstücke in die Polymerlösung eingetaucht, wobei in einem anschließenden Schritt das Lösemittel an Luft oder in einer Vakuumkammer verdampft wurde. Hierdurch hat sich die schützende Polymerschicht auf der Oberfläche des Werkstücks ausgebildet. Bei der Beschichtung von Magnesiumproben hat sich gezeigt, dass die Proben eine signifikant erhöhte Korrosionsbeständigkeit im Vergleich zu unbehandelten Proben aufwiesen. Insbesondere wurde eine konstant bleibende Korrosionsschutzwirkung auf das Werkstück auch bei Klimawechseln und Chloridbelastung (wie z.B. Salzsprühtests etc.) auch nach mehreren Tagen (30 Tage) beobachtet.

Des Weiteren wird ein Verfahren zur Kontrolle der Korrosionsrate von einer aus einem Werkstück aus Magnesium oder einer Magnesiumlegierung gebildeten, vorzugsweise selbstauflösenden, Bio-Komponente vorgeschlagen, wobei eine Beschichtungslage auf dem Werkstück gemäß dem Verfahren nach einem der Ansprüche 1 bis 10 ausgebildet ist oder wird, und wobei die Korrosionsrate über die Porosität der, vorzugsweise selbstauflösenden, Biokomponente, z.B. Implantaten kontrolliert wird. Durch die aufgebrachte Beschichtungslage wird eine Beschichtung mit einer vorbestimmten, definierten Korrosionsrate, insbesondere von unter 1,0 mm/Jahr bei einer reinen Magnesiumlegierung, gemessen mittels eines Salzsprühtests nach DIN 50021, aufgetragen, so dass sich die in einem menschlichen Körper implantierte Biokomponente nach dem Ausheilen beispielsweise einer Fraktur selbst auflöst.

## Patentansprüche

1. Verfahren zum Behandeln eines Werkstücks aus Magnesium oder einer Magnesiumlegierung, **dadurch gekennzeichnet, dass** das Werkstück mit einer Polymerlösung benetzt oder versehen wird, wobei die Polymerlösung Polyetherimid und ein Lösemittel enthält,
wobei als ein mit Wasser mischbares Lösemittel Dimethylacetamid (DMAc) und/oder Dimethylformamid (DMF) und/oder N-Methylpyrrolidon (NMP) und/oder gamma-Butyrolacton (GBL) oder wobei als ein mit Wasser nicht-mischbares Lösemittel Dichlormethan und/oder Chloroform und/oder 1,2-Dichlorethan eingesetzt wird,
wobei nach Trocknung des Werkstücks, vorzugsweise nach Herausziehen des Werkstücks aus der oder nach Benetzung des Werkstücks mit der Polymerlösung, eine korrosionsbeständige Beschichtungslage auf der Oberfläche des Werkstücks ausgebildet ist oder wird.

2. Verfahren nach Anspruch 1, **dadurch gekennzeichnet, dass** das Werkstück in die Polymerlösung eingetaucht oder mit der Polymerlösung besprüht wird.

3. Verfahren nach Anspruch 1 oder 2, **dadurch gekennzeichnet, dass** die Oberfläche des Werkstücks vor dem Benetzen gereinigt wird oder ist.

4. Verfahren nach Anspruch 1 oder 2, **dadurch gekennzeichnet, dass** die Oberfläche des Werkstücks vor dem Benetzen ungereinigt ist.

5. Verfahren nach einem der Ansprüche 1 bis 4, **dadurch gekennzeichnet, dass** vor dem Benetzen mit der Polyetherimid-Polymerlösung die Oberfläche des Werkstücks mit einer Konversionsschicht oder einer Haftschicht versehen wird.

6. Verfahren nach einem der Ansprüche 1 bis 5, **dadurch gekennzeichnet, dass** die Konzentration von Polyetherimid in der Polymerlösung zwischen 0,5 bis 20 Gew.% beträgt.

7. Verfahren nach einem der Ansprüche 1 bis 6, **dadurch gekennzeichnet, dass** die aufgebrachte Beschichtungslage auf dem Werkstück derart ausgebildet ist oder wird, dass das Werkstück bei einer mechanischen Krafteinwirkung auf das Werkstück an der Stelle der Krafteinwirkung durch die Beschichtungslage gegen Korrosion geschützt bleibt.

8. Verfahren nach einem der Ansprüche 1 bis 7, **dadurch gekennzeichnet, dass** die Polymerlösung auf ein mit einer defekten Korrosionsschicht versehenes Werkstück aufgebracht wird, so dass die defekte Stelle der Korrosionsschicht mit einer Beschichtungslage oder Beschichtung versehen wird.

9. Verfahren nach einem der Ansprüche 1 bis 8, **dadurch gekennzeichnet, dass** die Haftung der Beschichtungslage größer als die Haftung einer Beschichtungslage aus Acrylat, insbesondere bei gleicher Beschichtungsdicke, ist.

10. Verfahren nach einem der Ansprüche 1 bis 9, **dadurch gekennzeichnet, dass** die Polymerlösung Partikel oder Inhibitoren oder therapeutische oder medikamentöse Wirkstoffe enthält, so dass Partikel oder Inhibitoren oder therapeutische oder medikamentöse Wirkstoffe in die Beschichtungslage eingebaut oder eingebracht oder aufgebracht werden.

11. Verfahren zur Kontrolle der Korrosionsrate von einer aus einem Werkstück aus Magnesium oder einer Magnesiumlegierung gebildeten, vorzugsweise selbstauflösenden, Bio-Komponente, wobei eine Beschichtungslage auf dem Werkstück gemäß dem Verfahren nach einem der Ansprüche 1 bis 10 ausgebildet ist oder wird, und wobei die Korrosionsrate über eine Porosität der Biokomponente kontrolliert wird.

## Claims

1. A method of treating a workpiece of magnesium or a magnesium alloy, **characterised in that** the workpiece is wetted or provided with a polymer solution, wherein the polymer solution includes polyetherimide and a solvent, wherein dimethylacetamide (DMAc) and/or dimethylformamide (DMF) and/or N-methylpyrrolidone (NMP) and/or gamma-butyrolactone (GBL) is used as a solvent which is miscible with water or wherein dichloromethane and/or chloroform and/or 1,2-dichloroethane is used as a solvent which is immiscible with water, wherein after drying of the workpiece, preferably after withdrawing the workpiece from or after the wetting process of the workpiece with the polymer solution, a corrosion-resistant coating layer is formed on the surface of the workpiece.

2. A method as claimed in claim 1, **characterised in that** the workpiece is dipped into the polymer solution or is sprayed with the polymer solution.

3. A method as claimed in claim 1 or 2, **characterised in that** the surface of the workpiece is cleaned before the wetting process.

4. A method as claimed in claim 1 or 2, **characterised in that** the surface of the workpiece is uncleaned before the wetting process.

5. A method as claimed in one of claims 1 to 4, **characterised in that** before the wetting process with the polyetherimide polymer solution, the surface of the workpiece is provided with a conversion layer or an undercoat.

6. A method as claimed in one of claims 1 to 5, **characterised in that** the concentration of polyetherimide in the polymer solution is between 0.5 to 20wt.%.

7. A method as claimed in one of claims 1 to 6, **characterised in that** the applied coating layer is formed on the workpiece such that when a mechanical force acts on the workpiece, the workpiece remains protected by the coating layer at the position of the action of the force.

8. A method as claimed in one of claims 1 to 7, **characterised in that** the polymer solution is applied to a workpiece provided with a defective corrosion layer so that the defective position of the corrosion layer is provided with a coating layer or coating.

9. A method as claimed in one of claims 1 to 8, **characterised in that** the adhesion of the coating layer is greater than the adhesion of a coating layer of acrylate, particularly with the same coating thickness.

10. A method as claimed in one of claims 1 to 9, **characterised in that** the polymer solution includes particles or inhibitors or therapeutic or medicinal active ingredients so that particles or inhibitors or therapeutic or medicinal active components are incorporated or introduced or applied into the coating layer.

11. A method of monitoring the corrosion rate of a biocomponent, preferably of soluble type, formed from a workpiece of magnesiumor magnesium alloy, wherein a coating layer is formed on the workpiece with the method as claimed in one of claims 1 to 10 and wherein the corrosion rate is monitored by way of a porosity of the biocomponent.

## Revendications

1. Procédé de traitement d'une pièce usinée en magnésium ou en alliage de magnésium, **caractérisé en ce que** la pièce est mouillée par ou dotée d'une solution de polymère, la solution de polymère contenant un polyéther-imide et un solvant,
dans lequel on utilise comme solvant miscible à l'eau du diméthyle acétamide (DMAc), et/ou du diméthyle formamide (DMF) et/ou de la N-méthyl-pyrrolidone (NMP) et/ou de la gamma butyrolactone (GBL), ou dans lequel on utilise comme solvant non miscible à l'eau du dichlorométhane et/ou du chloroforme et/ou du 1,2-dichloréthane,
dans lequel, après séchage de la pièce usinée, de préférence après l'extraction de la pièce usinée de la solution de polymère ou après le mouillage de la pièce usinée par la solution de polymère, une couche de revêtement résistant à la corrosion est formée ou se forme à la surface de la pièce usinée.

2. Procédé selon la revendication 1, **caractérisé en ce que** la pièce usinée est plongée dans la solution de polymère ou est aspergée de la solution de polymère.

3. Procédé selon la revendication 1 ou 2, **caractérisé en ce que** la surface de la pièce usinée est nettoyée avant le mouillage ou **en ce qu'**on la nettoie.

4. Procédé selon la revendication 1 ou 2, **caractérisé en ce que** la surface de la pièce usinée n'est pas nettoyée avant le mouillage.

5. Procédé selon l'une quelconque des revendications 1 à 4, **caractérisé en ce qu'**avant le mouillage par la solution de polymère polyéther-imide, la surface de la pièce usinée est dotée d'une couche de conversion ou d'une couche d'adhérence.

6. Procédé selon l'une quelconque des revendications 1 à 5, **caractérisé en ce que** la concentration du polyéther-imide dans la solution de polymère est comprise entre 0,5 et 20 % en poids.

7. Procédé selon l'une quelconque des revendications 1 à 6, **caractérisé en ce que** la couche de revêtement appliquée se forme ou est formée sur la surface de la pièce usinée de telle sorte que, lors de l'effet d'une force mécanique agissant sur la pièce usinée, la pièce usinée reste protégée de la corrosion par la couche de revêtement à l'endroit de l'effet de ladite force mécanique.

8. Procédé selon l'une quelconque des revendications 1 à 7 **caractérisé en ce que** la solution de polymère est appliquée sur une pièce usinée dotée d'une couche de corrosion défectueuse, de telle sorte que l'endroit défectueux de la couche de corrosion est doté d'une couche de revêtement ou d'un revêtement.

9. Procédé selon l'une quelconque des revendications 1 à 8, **caractérisé en ce que** l'adhérence de la couche de revêtement est plus grande que l'adhérence d'une couche de revêtement en acrylate, en particulier pour la même épaisseur de revêtement.

10. Procédé selon l'une quelconque des revendications 1 à 9, **caractérisé en ce que** la solution de polymère contient des particules ou des inhibiteurs ou des principes actifs thérapeutiques ou médicamenteux, de telle sorte que des particules ou des inhibiteurs ou des principes actifs thérapeutiques ou médicamenteux sont incorporés ou introduits ou appliqués dans ou sur la couche de revêtement.

11. Procédé pour le contrôle du taux de corrosion d'un composant biologique formé d'une pièce usinée en magnésium ou en alliage de magnésium, de préférence autodissoluble, sur laquelle est formée ou se forme une couche de revêtement selon l'une quelconque des revendications 1 à 10, et pour laquelle le taux de corrosion est contrôlé par l'intermédiaire d'une porosité du composant biologique.
